# EUROPÄISCHE PATENTANMELDUNG

(11) **EP 1 902 753 A1**
(43) Veröffentlichungstag der Anmeldung: **26.03.2008**
(21) Anmeldenummer: 06021937.5
(22) Anmeldetag: 19.10.2006
(51) Int. Cl.: A61Q 11/00, A61K 8/97, A61K 8/92, A61K 8/25

(54) **Spülung für den Mundinnenraum**

(30) Priorität: 19.09.2006 EP 06019584
(71) Anmelder: Tenspolde, Thomas, Dr., 47800 Krefeld (DE)
(72) Erfinder: Tenspolde, Thomas, Dr., 47800 Krefeld (DE)
(74) Vertreter: DR. STARK & PARTNER

(57) **Zusammenfassung**

Die Erfindung betrifft eine Spülung für den Mundinnenraum aus pflanzlichem Fett, insbesondere pflanzlichem Öl, wie z. B. Sonnenblumenöl, die im Mundinnenraum geschlürft, gespült und/oder gegurgelt bzw. durch die Zahnzwischenräume gezogen wird und anschließend ausgespukt wird.

Damit auch insbesondere bei stärkeren Erkrankungen, beispielsweise des Zahnfleisches, wie z. B. Zahnfleischrückbildung oder dergleichen, eine ausreichende Wirkung erzielbar ist, soll der Spülung Aloe Vera Saft bzw. Aloe Vera Gel, insbesondere in Form eines 10-fach-Konzentrates, vorzugsweise in einem Volumenverhältnis von wenigstens 1:100 (Aloe Vera:pflanzliches Fett), zugesetzt sein.

## Beschreibung

Die Erfindung betrifft eine Spülung für den Mundinnenraum aus pflanzlichem Fett, insbesondere pflanzlichem Öl, wie z. B. Sonnenblumenöl, die im Mundinnenraum geschlürft, gespült und/oder gegurgelt bzw. durch die Zahnzwischenräume gezogen wird und anschließend ausgespukt wird. Unter Mundinnenraum wird vorliegend die Mundhöhle und/oder der Rachenraum verstanden.

Aus der Praxis sind derartige Spülungen bekannt, bei denen ca. 5 ml bis 15 ml der Spülung, d. h. die Menge eines Teelöffels bis hin zur Menge eines Esslöffels, in den Mundinnenraum gegeben wird und dann für einen Zeitraum von fünf bis maximal zwanzig Minuten durch Saugen, Spülen, Gurgeln oder durch die Zähne Ziehen hin und her bewegt wird. Dabei vermischt sich das Öl mit Speichel und wird nach dem Spülen ausgespuckt. Anschließend wird der Mundinnenraum mehrmals gründlich gespült und die Zähne und insbesondere auch die Zunge mit der Zahnbürste gereinigt. Dieser Vorgang ist als Ölziehen, Ölkauen oder Ölschlürfen bekannt.

Nachteilig hierbei ist, dass zwar ein positiver Effekt durch das Spülen eintritt, jedoch insbesondere bei stärkeren Erkrankungen, beispielsweise des Zahnfleisches, wie z. B. Zahnfleischrückbildung oder dergleichen, die erzielbare Wirkung nicht ausreicht.

Erfindungsgemäß soll daher der Spülung Aloe Vera Saft bzw. Aloe Vera Gel, insbesondere in Form eines 10-fach-Konzentrates, vorzugsweise in einem Volumenverhältnis von wenigstens 1:100 (Aloe Vera:pflanzliches Fett), d. h. ein Teil Aloe Vera Saft bzw. Aloe Vera Gel, insbesondere in Form eines 10-fach-Konzentrates, auf 100 Teile Pflanzenfett, insbesondere Pflanzenöl, zugesetzt sein. Hierdurch werden aufgrund der in dem Aloe Vera Saft bzw. Gel enthaltenen Zuckerart Acemannan die Makrophagen (Fresszellen), die Monozyten, die Antikörper und die T-Killerzellen aktiviert.

Dadurch erfolgt eine Abwehr bzw. Vernichtung von Antigenen, was eine Regenerierung der Mundschleimhaut sowie eine Straffung der Mundschleimhaut bewirkt. Weiterhin werden Zahnfleischtaschen verringert und es erfolgt eine deutliche Verringerung der Blutungsneigung des Zahnfleischs.

Vorzugsweise kann das Volumenverhältnis wenigstens 1:10, insbesondere 1:4 bis 1:1, betragen und es sind auch Volumenverhältnisse von höchstens 10:1, insbesondere 4:1 bis 2:1 möglich.

Erfindungsgemäß kann die Spülung vor Gebrauch statt einer flüssigen, insbesondere zäh- bzw. dickflüssigen Konsistenz einen nicht-flüssigen, insbesondere festen Zustand haben, so dass sowohl die Aufbewahrung und Handhabung als auch die Portionierung einfacher und praxisgerechter erfolgen kann. Insoweit kann eine Portion der Spülung ähnlich einer aus der Gastronomie bekannten Portionsbutter einzeln verpackt sein und wird erst im Mund verflüssigt.

Vorzugsweise kann der Aloe Vera Saft bzw. das Aloe Vera Gel aus der Sorte Aloe Vera Barbadensis Miller gewonnen sein.

Vorteilhafterweise kann die Spülung weitere medizinisch und/oder chemisch wirksame Bestandteile aufweisen, so dass zusätzliche positive Effekte erzielbar sind.

Dabei kann die Spülung als weiteren medizinisch und/oder chemisch wirksamen Bestandteil SiO₂ aufweisen, wobei durch das Siliciumdioxid eine verstärkte Sauerstoff-Anreicherung in den Mitochondrien bewirkt wird, was eine verbesserte Reduplikationsrate bewirkt, so dass die Neubildung der Schleimhaut verbessert wird.

Auch kann die Spülung als weiteren medizinisch und/oder chemisch wirksamen Bestandteil SiO₄, d. h. Zeolith, insbesondere einen nanoskaliges Zeolith, aufweisen, wodurch freie Radikale gebunden und dann entfernt werden. Durch die Teilchengröße im Nanopartikelbereich ist eine besonders große Reaktionsoberfläche gegeben und die Zeolithpartikel können jeden noch so kleinen Zwischenraum erreichen.

Weiterhin kann die Spülung als weiteren Bestandteil zumindest einen geschmacksverbessernden Stoff, wie z. B. ein ätherisches Öl, z. B. Menthol oder Eukalyptus, einen Süßstoff oder einen Aromastoff (natürlich, naturidentisch oder künstlich), aufweisen, so dass die Anwendung der Spülung durch den Benutzer angenehmer ist.

## Patentansprüche

1. Spülung für den Mundinnenraum aus pflanzlichem Fett, insbesondere pflanzlichem Öl, wie z. B. Sonnenblumenöl, die im Mundinnenraum geschlürft, gespült und/oder gegurgelt bzw. durch die Zahnzwischenräume gezogen wird und anschließend ausgespukt wird, **dadurch gekennzeichnet, dass** Aloe Vera Saft bzw. Aloe Vera Gel, insbesondere in Form eines 10-fach-Konzentrates, vorzugsweise in einem Volumenverhältnis von wenigstens 1:100 (1 Teil Aloe Vera zu 100 Teilen pflanzlichem Fett), zugesetzt ist.

2. Spülung nach dem vorhergehenden Anspruch, **dadurch gekennzeichnet, dass** das Volumenverhältnis wenigstens 1:10, insbesondere 1:4 bis 1:1, beträgt.

3. Spülung nach einem der vorhergehenden Ansprüche, **dadurch gekennzeichnet, dass** die Spülung vor Gebrauch einen nicht-flüssigen, insbesondere festen Zustand hat.

4. Spülung nach einem der vorhergehenden Ansprüche, **dadurch gekennzeichnet, dass** der Aloe Vera Saft bzw. das Aloe Vera Gel aus der Sorte Aloe Vera Barbadensis Miller gewonnen ist.

5. Spülung nach einem der vorhergehenden Ansprüche, **dadurch gekennzeichnet, dass** die Spülung weitere medizinisch und/oder chemisch wirksame Bestandteile aufweist.

6. Spülung nach Anspruch 5, **dadurch gekennzeichnet, dass** die Spülung als weiteren medizinisch und/oder chemisch wirksamen Bestandteil SiO₂ aufweist.

7. Spülung nach Anspruch 5 oder 6, **dadurch gekennzeichnet, dass** die Spülung als weiteren medizinisch und/oder chemisch wirksamen Bestandteil SiO₄ aufweist.

8. Spülung nach einem der vorhergehenden Ansprüche, **dadurch gekennzeichnet, dass** die Spülung als weiteren Bestandteil zumindest einen geschmacksverbessernden Stoff, wie z. B. ein ätherisches Öl, einen Süßstoff oder einen Aromastoff, aufweist.
